# EUROPEAN PATENT SPECIFICATION

(11) **EP 2 680 916 B1**
(45) Date of publication and mention of the grant of the patent: **09.09.2015**
(21) Application number: 12706276.8
(22) Date of filing: 02.03.2012
(51) Int. Cl.: A61M 25/06

(54) **A MEDICAL DEVICE FOR INTRODUCING A CATHETER INTO A BLOOD VESSEL**
MEDIZINISCHE VORRICHTUNG ZUM EINFÜHREN EINES KATHETERS IN EIN BLUTGEFÄSS
DISPOSITIF MÉDICAL POUR INTRODUIRE UN CATHÉTER DANS UN VAISSEAU SANGUIN

(30) Priority: 04.03.2011 IT PD20110070
(43) Date of publication of application: 08.01.2014
(73) Proprietor: Leader Medica S.r.l., 35129 Padova (IT)
(72) Inventor: CERON, Mauro, I-46019 Viadana (MN) (IT); MINOZZI, Roberto, I-35030 Selvazzano Dentro (PD) (IT)
(74) Representative: Susanetto, Carlo
(86) International application number: PCT/EP2012/053618
(87) International publication number: WO 2012/119939

(56) References cited:
- WO-A1-2010/089727
- WO-A2-02/066093
- WO-A2-2008/137956
- US-A1- 2009 069 751

## Description

### Technical field

The present invention relates to a medical device for introducing a catheter into a blood vessel, having the features set out in the preamble of the main claim.

### Technological background

In medical technical field, there is known the need to insert a catheter inside an artery or a vein, in order, for example, to allow the rapid administration of medicaments or dialyzed blood inside the organism.

One of the techniques commonly used to introduce a catheter in an artery or a vein provides for the insertion into the blood vessel of a needle via the percutaneous route, through which there is first passed a guide wire which is then pushed inside the blood vessel to a desired extent. Subsequently, the hole formed by the needle is enlarged by means of an expanding device and, after the needle and expanding device have been disengaged, the catheter is introduced into the blood vessel along the guide wire.

Over the years, there have been developed suitable devices which are generally configured in a syringe-like manner, comprising a cylindrical tubular body in which there is slidingly engaged a retractable needle, whose movement is controlled by a slider which projects from the cylindrical tubular body.

Although such devices, an example of which is described in the international patent application WO01/78595, have undoubtedly simplified the procedure for inserting the catheter, they still have some disadvantages.

Other examples of devices according to the prior art are disclosed in WO 02/066093.

A first disadvantage involves the fact that the syringe-like device, which has to allow ready manipulation, has dimensions so as to prevent the needle from being introduced in a direction which is inclined as little as possible with respect to the vein or artery involved in the catheterization. This is caused mainly by the spatial requirement of the cylindrical tubular body.

A second disadvantage involves the fact that, in those devices, the needle has to be constantly held in position by the action of the operator when it projects from the cylindrical tubular body, otherwise it could readily slide backwards. Furthermore, a requirement which is always present in this field is that of ensuring the maximum level of safety of the operators so that the possibility that they might be pricked by the needle is prevented at all times. In particular, there is the requirement of providing first safety means which prevent accidental discharges of the needle before it is used, as well as second safety means which prevent, in an irreversible manner, the discharge of the needle after it has been used.

### Summary of the invention

The problem addressed by the present invention is to provide a medical device which is for the introduction of catheters into a blood vessel and which is structurally and functionally configured to overcome the limitations set out above with reference to the cited prior art.

In the context of that problem, an object of the invention is to provide a device which is readily able to be handled and yet has a minimal spatial requirement in order to allow the introduction of the needle in a direction which is as parallel as possible with respect to the blood vessel.

A second object is to provide a medical device which increases the level of safety for the operators.

This problem is solved and those objects are achieved by the present invention by means of a medical device for the introduction of a catheter which is constructed in accordance with the appended claims.

### Brief description of the drawings

The features and advantages of the invention will be appreciated more clearly from the detailed description of a preferred embodiment thereof, which is illustrated by way of a non-limiting example with reference to the appended drawings, in which:
- Figure 1 is a perspective view from above and from behind of a medical device for the introduction of a catheter realised according to the present invention, in a first non-operative position,
- Figure 2 is a perspective view from above and from behind of the medical device of Figure 1 in an operative position,
- Figure 3 is a view, drawn to an enlarged scale, of a detail of Figure 2,
- Figure 4 is a perspective view from above and from behind of the medical device of Figure 1 in a second non-operative position,
- Figure 5 is a longitudinal section of the device of Figure 4,
- Figure 6 is a longitudinal section along the line VI-VI of Figure 5.

### Preferred embodiment of the invention

In the Figures, there is generally designated 1 a medical device which is for the introduction of a catheter and which is realised according to the present invention. The device 1 comprises a tubular body 2 which extends in a prevailing longitudinal direction X and in which there is defined a single chamber 3 which is delimited by a wall having a substantially constant thickness and which is open at longitudinally opposite ends at a front end 4 and a rear end 5 of the tubular body 2.

The tubular body 2 further has, in the region of the chamber 3, a cross-section of non-circular form, generally elliptical, in which there are identified a major axis A and a minor axis B which are substantially perpendicular relative to each other and which intersect with the longitudinal axis X.

Preferably, with reference to a total length of the tubular body 2 of from approximately 150 mm to approximately 180 mm, the major axis A which can be identified with the width of the tubular body 2, has a dimension of from 8 to 20 mm, preferably of 12.5 mm, whilst the minor axis B which can be identified with the height of the tubular body 2 has a dimension of from 5 to 15 mm, preferably of approximately 8.5 mm.

In particular, it is preferable for the ratio between the dimensions of the major axis A and the minor axis B to be from 1.2 to 2, preferably to be of approximately 1.5.

The front end 4 of the tubular body 2 is advantageously formed by an expanding member 6 of frustoconical shape which is hollow internally.

There is connected to the tubular body 2 a retractable needle 10 which extends longitudinally and which is also of tube-like shape, being hollow internally, and is open both at a front end 11 thereof, of pointed shape, and at a rear end 12 thereof.

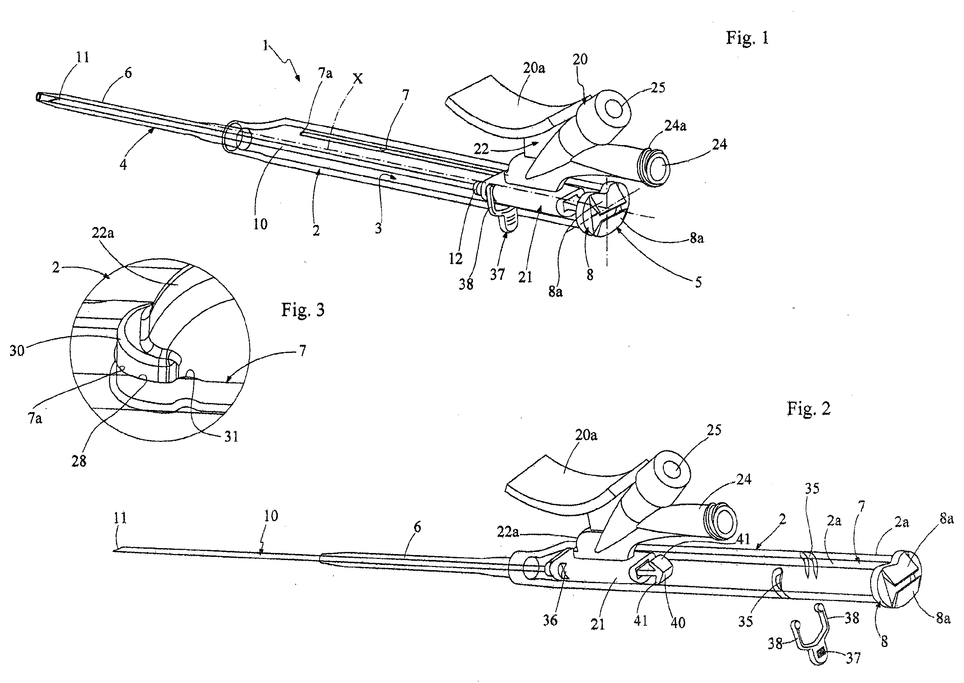

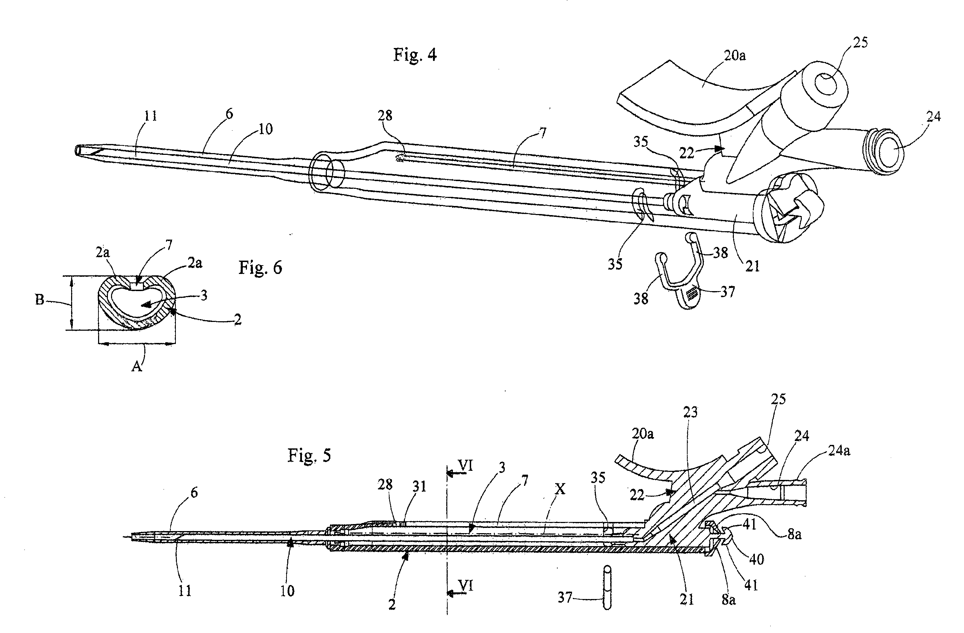

The needle 10 can be moved inside the chamber 3 between an operative position (see Figure 2), in which the needle 10 projects, at least at the front end 11 extending through the expanding member 6, from the front end 4 of the tubular body 2, and a non-operative position (Figures 1 and 4), in which the needle 10 is housed inside the tubular body 2, partially inside the chamber 3 and partially inside the expanding member 6, without projecting therefrom. The needle 10 is further fixed, in the region of the rear end 12 thereof, to a slider 20 which is engaged with a capability to slide on the tubular body 2. The slider 20 comprises an internal portion 21, accommodated in the chamber 3 of the tubular body 2, and an external portion 22 which projects from the tubular body 2 parallel with the minor axis B.

The needle 10 preferably does not extend coaxially with respect to the tubular body 2 but, advantageously, it is located at the opposite side to the slider 20 with respect to the longitudinal axis X of the tubular body 2, adjoining as closely as possible the wall of the tubular body 2 opposite the external portion 22 of the slider 20.

The external portion 22 projects from the tubular body 2 through a longitudinal slot 7 which is formed in the tubular body 2 and which thus acts as a guide during the movement of the slider 20.

Preferably, at the slot 7, the tubular body 2 is lowered with respect to the zones of the tubular body 2a which flank the slot 7, thereby obtaining another reduction in the dimension along the minor axis B (height) of the tubular body 2.

A support element 20a is provided on the external portion 22 of the slider 20 in order to allow movement of the slider 20, and consequently of the needle 10 which is connected thereto, away from and towards the non-operative position.

There is internally formed in the slider 20 a duct 23 which is open on one side in the region of the rear end 12 of the needle 10 and on the other side with a first and second mouth 24 and 25 which are both formed in the external portion 22 of the slider 20. In that manner, the duct 23 is open at the outer side of the slider 20 only in the region of the external portion 22.

The first mouth 24 extends in a substantially longitudinal manner and is provided with a standard attachment for connection to a suction syringe, in particular it is provided with an attachment 24a of the Luer type. In that manner, the optional syringe which is connected to the slider 20 is, during use, substantially parallel with the tubular body 2.

The second mouth 25 extends in an incident manner with respect to the longitudinal direction X and is arranged in particular for the entry of a guide wire.

The slot 7 is closed both at the rear end thereof for the provision of a cap 8 which is mounted at the rear end 5 of the tubular body 2 and at the front end 7a thereof.

The cap 8 acts as a stop member which is capable of checking the travel of the slider 20 when it is moved towards the rear end 5 of the tubular body 2, preventing the slider 20 from being discharged from the body.

The cap 8 is open in a central zone thereof and comprises a pair of walls 8a which extend towards the above-mentioned central zone at the opposing sides of the cap 8. The walls 8a extend in a slightly inclined manner away from the tubular body 2 without touching each other and advantageously have a profile having a cross-section which tapers from the base in contact with the tubular body towards the free base so as to allow limited pivoting thereof in the region of the free base.

The device 1, in the region of the front end 4 of the tubular body 2, is provided with temporary holding means for the slider 20 in order to hinder the free sliding thereof with respect to the tubular body 2 when the needle 10 is moved into the operative position.

The temporary holding means for the slider 20 comprise a seat 28 which is formed at the front end 7a of the slot 7 and snap coupling means which are provided between the slider 20 and the seat 28.

In particular, the snap coupling means comprise an enlarged head 30 which is defined at a front 22a of the external portion 22 which can slide in the slot 7, and a narrowed portion 31 which is provided in the slot 7 for delimiting the seat 28.

In a construction variant of the device 1, which is not illustrated, the temporary holding means may comprise a friction element which is provided inside the tubular body 2 at the end of the chamber 3 which faces the expanding element 6 so as to produce greater friction with the slider 20 with respect to the internal wall of the tubular body 2 and therefore to limit the slidability between the tubular body 2 and the slider 20 when it is moved into an operative position, with the needle 10 projecting from the expanding element 6. The friction element may be, for example, formed by the same internal wall of the tubular body 2, which wall is advantageously corrugated, or by a layer of material with a friction coefficient greater than that with which the tubular body is constructed.

The slider 20 is further movable along the slot 7 at the longitudinally opposite side in a first or in a second non-operative position.

In the first non-operative position, illustrated in Figure 1, the slider 20 is freely movable towards the operative position whilst, in the second non-operative position, illustrated in Figure 4, the slider 20 abuts the cap 8 and is irreversibly secured to the tubular body 2 in order to prevent any possible movement of the needle 10 towards the operative position.

In order to hold the slider 20 in the first non-operative position, the device 1 comprises removable locking means.

There is preferably provision for the removable locking means to be of the type having a pin 38 and for recesses, which are capable of being aligned when the slider 20 is moved into the first non-operative position and in which the pin can be removably received, to be formed in the tubular body 2 and the slider 20, respectively.

In detail, there is formed, on the tubular body 2, at the side opposed to the major axis A, a first pair of notches 35 which are parallel with the minor axis B whilst there is formed, on the internal portion 21 of the slider 20, on the side opposed to the major axis A, a second pair of notches 36 which are parallel with the minor axis B. The removable locking means comprise a fork 37, the prongs 38 of which form pins which are capable of engaging with both the pairs of notches 35, 36 when the slider is in the first non-operative position (see Figure 1).

In order to hold the slider 20 in the second non-operative position, the device 1 comprises irreversible locking means which comprise an anchor member 40 which extends from the internal portion 21 of the slider 20 in the opposite direction to the needle 10.

The anchor member 40 comprises a pair of teeth 41 which, when the slider 20 is moved into the second non-operative position, are capable of becoming engaged with the walls 8a of the cap 8 at the side opposite the tubular body 2. The teeth 41 are advantageously inclined towards the front end 4 of the tubular body 2 so as to form an undercut so that the anchor member 40 cannot be disengaged from the cap 8 unless the member itself or the walls 8a are broken.

The medical device 1 is supplied in the first non-operative position illustrated in Figure 1, in which the needle 10 is completely retracted inside the tubular body 2 and the fork 37 is inserted in engagement in the notches 35, 36 in order to hold the slider 20 in that position, thereby avoiding the undesirable possibility that the needle 10 may be accidentally moved outside the tubular body 2.

At the time of its use, the fork 37 is removed and, by the support 20a being acted upon, the slider 20 and consequently also the needle 10 are moved into an operative position, in particular the slider 20 is urged as far as the front end 7a of the slot 7, where the enlarged head 30 is received in the seat 28 after passing the narrowed portion 31.

Normally, a syringe is connected to the first mouth 24 of the slider 20 by means of the Luer attachment provided thereon, so that, when the needle 10 is inserted in the body of a patient, it is possible to verify whether the needle 10 has reached the vein or artery in which the catheter has to be inserted, by means of intake of a reduced quantity of blood through the needle 10 and the duct 23.

It shall be noted that, owing to the special configuration of the tubular body 2, in particular owing to the reduced dimension of the minor axis B and the lowered positioning of the needle 10 with respect to the axis X, the needle 10 can be inserted in the manner that is inclined as little as possible with respect to the vein or artery. Furthermore, once moved into an operative position, the needle 10 can be moved back into a non-operative position (in the first or second position) simply by applying a given force to the slider 20 which allows the discharge of the enlarged head 30 out of the seat 28. In that manner, accidental movements of the needle are prevented or at least greatly limited. Once the desired blood vessel has been reached, a guide wire is introduced therein through the second mouth 25, the duct 23 and the needle 10 and, by means of the expanding member 6 provided at the front end 4 of the tubular body 2, the hole formed by the needle 10 can be advantageously enlarged.

At the end of this operation, the device 1 may be removed, leaving in position the guide wire for the insertion of the catheter which is carried out in accordance with usual methods.

The needle 10 can then be moved into the second non-operative position with the slider abutting the cap 8 and the anchor member 40 engaged in a non-removable manner with the walls 8a.

The present invention therefore solves the problem set out above with reference to the cited prior art, affording at the same time a number of other advantages, including an increased level of safety of the device before, during and after its use.

## Claims

1. A medical device for introducing a catheter, comprising:
- a tubular body (2) which extends in a longitudinal direction (X) and in which a single chamber (3) open at the longitudinally opposite ends is defined,
- a longitudinally extending retractable needle (10) connected to the tubular body and movable inside the chamber between an operative position, in which the needle projects from the tubular body, and at least one non-operative position, in which the needle is housed inside the tubular body,
- a slider (20) secured to the needle (10) and engaged on the tubular body, with the capability to slide in the chamber, the slider projecting at least partially from the tubular body to bring about the movement of the needle,
**characterized in that** the tubular body has, at the chamber, a non-circular cross-section on which are identified a major axis (A) and a minor axis (B) which are substantially perpendicular to each other, the slider projecting from the tubular body parallel with the minor axis.

2. A medical device according to claim 1, wherein the tubular body (2) comprises, at a front end (4) thereof, a frustoconical expanding member (6) through which the needle (10) passes and inside which the needle is partially accommodated when in the non-operative position.

3. A medical device according to claim 1 or 2, wherein the ratio between the major axis (A) and the minor axis (B) is from 1.2 to 2.

4. A medical device according to claim 3, wherein the major axis (A) has a dimension of from 8 to 20 mm and the minor axis (B) has a dimension of from 5 to 15 mm.

5. A medical device according to any one of the preceding claims, wherein the slider (20) comprises an internal portion (21) accommodated in the tubular body, and an external portion (22) projecting from the tubular body, and the needle (10) extends in such a manner that it is parallel with and spaced from a longitudinal axis of the chamber and is located at the opposite side to the external portion (22) of the slider (20).

6. A medical device according to any one of the preceding claims, wherein the needle (10) is internally hollow and open at a front end (4) thereof, and a duct (23) which opens, on one side, to the inside of the needle and, on the other side, to the outside of the device, is formed in the slider.

7. A medical device according to claim 6, wherein the slider comprises an internal portion (21) accommodated in the tubular body, and an external portion (22) projecting from the tubular body, the duct (23) being open to the outside at the external portion only.

8. A medical device according to claim 7, wherein the duct (23) is open at a first mouth (24) which extends substantially longitudinally and which is arranged for the attachment of a suction syringe.

9. A medical device according to claim 7 or 8, wherein the duct (23) is open at a second mouth (25) which extends in an incident manner with respect to the longitudinal direction and which is arranged for the entry of a guide wire.

10. A medical device according to any one of the preceding claims, wherein temporary holding means are provided at the front end (4) of the tubular body for temporarily holding the slider (20) in order to hinder the sliding of the slider with respect to the tubular body when the needle is moved into the operative position.

11. A medical device according to claim 10, wherein the temporary holding means comprise a friction member arranged between the tubular body and the slider.

12. A medical device according to claim 10, wherein the tubular body has a longitudinal slot (7) in which the slider is slidingly engaged, the slot being closed at a front end (7a) thereof, and the temporary holding means comprise a seat (28) formed at the front end of the slot and means (30, 31) for snap coupling between the slider and the seat.

13. A medical device according to claim 12, wherein, at the slot (7), the tubular body is lowered with respect to the zones (2a) of the tubular body which flank the slot.

14. A medical device according to any one of the preceding claims, wherein the slider is movable into a first non-operative position in which the slider can be moved towards the operative position again, and into a second non-operative position which is irreversible and in which the slider is anchored securely to the tubular body in order to prevent any possible movement of the slider towards the operative position.

15. A medical device according to claim 14, wherein:
- the slider comprises an internal portion (21) accommodated in the tubular body and an external portion (22) projecting from the tubular body,
- there is provided on the tubular body, at the end remote from the needle, a stop member (8) capable of stopping the travel of the slider when the latter is moved into the second non-operative position and preventing it from coming out of the tubular body,
- there extends, from the internal portion of the slider in the opposite direction to the needle, an anchor member (40) capable of engaging irreversibly on the tubular body (2) or on the stop member (8) when the slider is moved into the second non-operative position against the stop member.

16. A medical device according to claim 14 or 15, wherein removable locking means are provided to hold the slider in the first non-operative position.

17. A medical device according to claim 16, wherein there are provided on the tubular body and on the slider respective recesses (35, 36) which are capable of being aligned when the slider is moved into the first non-operative position, and the removable locking means comprise a pin (38) capable of engaging removably in the aligned recesses.

18. A medical device according to claim 17, wherein the recesses comprise a first pair of notches (35) parallel with the minor axis (B) and formed in the tubular body on the side opposed to the major axis (A), and a second pair of notches (36) parallel with the minor axis and formed in the slider on the side opposed to the major axis, and the removable locking means comprise a fork (37), the prongs (38) of which form respective pins capable of engaging in the notches when the slider is in the first non-operative position.

## Patentansprüche

1. Medizinische Vorrichtung zum Einführen eines Katheters, umfassend:
- einen rohrförmigen Körper (2), der sich in Längsrichtung (X) erstreckt und in dem eine einzige Kammer (3) definiert ist, die an den in Längsrichtung gegenüberliegenden Enden offen ist,
- eine sich in Längsrichtung erstreckende rückziehbare Nadel (10), die mit dem rohrförmigen Körper verbunden ist und im Innern der Kammer zwischen einer Arbeitsstellung, bei der die Nadel aus dem rohrförmigen Körper vorragt, und zumindest einer Nicht-Arbeitsstellung beweglich ist, bei der die Nadel im Innern des rohrförmigen Körpers untergebracht ist,
- einen Schieber (20), der an der Nadel (10) befestigt ist und mit dem rohrförmigen Körper in der Kammer verschiebbar im Eingriff steht, wobei der Schieber zumindest teilweise aus dem rohrförmigen Körper vorragt, um die Bewegung der Nadel zu bewirken,
**dadurch gekennzeichnet, dass** der rohrförmige Körper in der Kammer einen unrunden Querschnitt aufweist, an dem eine Hauptachse (A) und eine Nebenachse (B) bestimmt sind, die im Wesentlichen senkrecht zueinander verlaufen, wobei der Schieber aus dem rohrförmigen Körper parallel zur Nebenachse vorragt.

2. Medizinische Vorrichtung nach Anspruch 1, wobei der rohrförmige Körper (2) an dessen Vorderende (4) ein sich kegelstumpfförmig erstreckendes Element (6) aufweist, durch das die Nadel (10) verläuft und in dessen Innern die Nadel in der Nicht-Arbeitsstellung teilweise untergebracht ist.

3. Medizinische Vorrichtung nach Anspruch 1 oder 2, wobei das Verhältnis zwischen der Hauptachse (A) und der Nebenachse (B) von 1,2 bis 2 reicht.

4. Medizinische Vorrichtung nach Anspruch 3, wobei die Hauptachse (A) eine Abmessung von 8 bis 20 mm aufweist und die Nebenachse (B) eine Abmessung von 5 bis 15 mm aufweist.

5. Medizinische Vorrichtung nach einem der vorhergehenden Ansprüche, wobei der Schieber (20) einen Innenbereich (21), der im rohrförmigen Körper untergebracht ist, und einen Außenbereich (22) aufweist, der aus dem rohrförmigen Körper vorragt, und sich die Nadel (10) derart erstreckt, dass diese zu einer Längsachse der Kammer parallel und von dieser beabstandet verläuft und an der zum Außenbereich (22) des Schiebers (20) gegenüberliegenden Seite angeordnet ist.

6. Medizinische Vorrichtung nach einem der vorhergehenden Ansprüche, wobei die Nadel (10) innen hohl und an deren Vorderende (4) offen ist, und eine Leitung (23), die sich auf einer Seite zur Innenseite der Nadel und an der anderen Seite zur Außenseite der Vorrichtung öffnet, im Schieber ausgebildet ist.

7. Medizinische Vorrichtung nach Anspruch 6, wobei der Schieber einen Innenbereich (21), der im rohrförmigen Körper untergebracht ist, und einen Außenbereich (22) aufweist, der aus dem rohrförmigen Körper vorragt, wobei die Leitung (23) nur am Außenbereich zur Außenseite offen ist.

8. Medizinische Vorrichtung nach Anspruch 7, wobei die Leitung (23) an einer ersten Öffnung (24) offen ist, die sich im Wesentlichen in Längsrichtung erstreckt und zur Befestigung einer Saugspritze eingerichtet ist.

9. Medizinische Vorrichtung nach Anspruch 7 oder 8, wobei die Leitung (23) an einer zweiten Öffnung (25) offen ist, die sich in Bezug auf die Längsrichtung einfallend erstreckt und zur Aufnahme eines Führungsdrahts eingerichtet ist.

10. Medizinische Vorrichtung nach einem der vorhergehenden Ansprüche, wobei eine temporäre Halteeinrichtung am Vorderende (4) des rohrförmigen Körpers zum temporären Halten des Schiebers (20) vorgesehen ist, um das Verschieben des Schiebers in Bezug auf den rohrförmigen Körper zu hemmen, wenn die Nadel in die Arbeitsposition bewegt wird.

11. Medizinische Vorrichtung nach Anspruch 10, wobei die temporäre Halteeinrichtung ein Reibelement aufweist, das zwischen dem rohrförmigen Körper und dem Schieber angeordnet ist.

12. Medizinische Vorrichtung nach Anspruch 10, wobei der rohrförmige Körper einen Längsschlitz (7) aufweist, mit dem der Schieber verschiebbar im Eingriff steht, wobei der Schlitz an dessen Vorderende (7a) geschlossen ist, und die temporäre Halteeinrichtung einen Sitz (28), der am Vorderende des Schlitzes ausgebildet ist, und eine Einrichtung (30, 31) für eine Schnappverbindung zwischen dem Schieber und dem Sitz aufweist.

13. Medizinische Vorrichtung nach Anspruch 12, wobei der rohrförmige Körper am Schlitz (7) in Bezug auf die Zonen (2a) des rohrförmigen Körpers abgesenkt ist, die den Schlitz flankieren.

14. Medizinische Vorrichtung nach einem der vorhergehenden Ansprüche, wobei der Schieber in eine erste Nicht-Arbeitsstellung, bei der der Schieber wieder zur Arbeitsstellung bewegt werden kann, und in eine zweite Nicht-Arbeitsstellung beweglich ist, die irreversibel ist und in welcher der Schieber am rohrförmigen Körper sicher verankert ist, um jede mögliche Bewegung des Schiebers zur Arbeitsstellung zu verhindern.

15. Medizinische Vorrichtung nach Anspruch 14, wobei:
- der Schieber einen Innenbereich (21), der im rohrförmigen Körper untergebracht ist, und einen Außenbereich (22) aufweist, der aus dem rohrförmigen Körper vorragt,
- ein Stoppelement (8) am rohrförmigen Körper am von der Nadel entfernten Ende vorgesehen ist, das ein Stoppen des Arbeitswegs des Schiebers ermöglicht, wenn dieser in die zweite Nicht-Arbeitsstellung bewegt wird, und verhindert, dass dieser aus dem rohrförmigen Körper austritt,
- ein Ankerelement (40) sich vom Innenbereich des Schiebers in die zur Nadel entgegengesetzte Richtung erstreckt, das einen irreversiblen Eingriff mit dem rohrförmigen Körper (2) oder dem Stoppelement (8) ermöglicht, wenn der Schieber gegen das Stoppelement in die zweite Nicht-Arbeitsstellung bewegt wird.

16. Medizinische Vorrichtung nach Anspruch 14 oder 15, wobei eine abnehmbare Verriegelungseinrichtung zum Halten des Schiebers in der ersten Nicht-Arbeitsstellung vorgesehen ist.

17. Medizinische Vorrichtung nach Anspruch 16, wobei am rohrförmigen Körper und am Schieber jeweilige Ausnehmungen (35, 36) vorgesehen sind, die ausrichtbar sind, wenn der Schieber in die erste Nicht-Arbeitsstellung bewegt wird, und die abnehmbare Verriegelungseinrichtung einen Stift (38) aufweist, der einen lösbaren Eingriff mit den ausgerichteten Ausnehmungen ermöglicht.

18. Medizinische Vorrichtung nach Anspruch 17, wobei die Ausnehmungen ein erstes Paar von Nuten (35), die parallel zur Nebenachse (B) verlaufen und im rohrförmigen Körper auf der zur Hauptachse (A) gegenüberliegenden Seite angeordnet sind, und ein zweites Paar von Nuten (36) aufweisen, die parallel zur Nebenachse verlaufen und im Schieber auf der zur Hauptachse gegenüberliegenden Seite ausgebildet sind, und die abnehmbare Verriegelungseinrichtung eine Gabel (37) aufweist, deren Zinken (38) jeweilige Stifte zum Eingriff in die Nuten bilden, wenn sich der Schieber in der ersten Nicht-Arbeitsstellung befindet.

## Revendications

1. Dispositif médical pour introduire un cathéter, comprenant :
un corps tubulaire (2) qui s'étend dans une direction longitudinale (X) et dans lequel est définie une chambre unique (3) ouverte au niveau des extrémités longitudinalement opposées,
une aiguille rétractable (10) s'étendant longitudinalement raccordée au corps tubulaire et mobile à l'intérieur de la chambre entre une position de fonctionnement dans laquelle l'aiguille fait saillie du corps tubulaire, et au moins une position de non fonctionnement, dans laquelle l'aiguille est logée à l'intérieur du corps tubulaire,
une glissière (20) fixée à l'aiguille (10) et mise en prise sur le corps tubulaire, avec la capacité de coulisser dans la chambre, la glissière faisant saillie au moins partiellement du corps tubulaire pour provoquer le mouvement de l'aiguille,
**caractérisé en ce que** le corps tubulaire a, au niveau de la chambre, une section transversale non circulaire sur laquelle sont identifiés un axe majeur (A) et un axe mineur (B) qui sont sensiblement perpendiculaires entre eux, la glissière faisant saillie du corps tubulaire parallèle à l'axe mineur.

2. Dispositif médical selon la revendication 1, dans lequel le corps tubulaire (2) comprend, au niveau de son extrémité avant (4), un élément d'expansion tronconique (6) à travers lequel passe l'aiguille (10) et à l'intérieur duquel l'aiguille est partiellement logée lorsqu'elle est dans la position de non fonctionnement.

3. Dispositif médical selon la revendication 1 ou 2, dans lequel le rapport entre l'axe majeur (A) et l'axe mineur (B) est de 1,2 à 2.

4. Dispositif médical selon la revendication 3, dans lequel l'axe majeur (A) a une dimension de 8 à 20 mm et l'axe mineur (B) a une dimension de 5 à 15 mm.

5. Dispositif médical selon l'une quelconque des revendications précédentes, dans lequel la glissière (20) comprend une partie interne (21) logée dans le corps tubulaire, et une partie externe (22) faisant saillie du corps tubulaire et l'aiguille (10) s'étend de sorte qu'elle est parallèle à et espacée d'un axe longitudinal de la chambre et est positionnée au niveau du côté opposé à la partie externe (22) de la glissière (20).

6. Dispositif médical selon l'une quelconque des revendications précédentes, dans lequel l'aiguille (10) est intérieurement creuse et ouverte au niveau de son extrémité avant (4), et un conduit (23) qui s'ouvre, d'un côté, vers l'intérieur de l'aiguille et de l'autre côté, vers l'extérieur du dispositif, est formé dans la glissière.

7. Dispositif médical selon la revendication 6, dans lequel la glissière comprend une partie interne (21) logée dans le corps tubulaire, et une partie externe (22) faisant saillie du corps tubulaire, le conduit (23) étant ouvert vers l'extérieur uniquement au niveau de la partie externe.

8. Dispositif médical selon la revendication 7, dans lequel le conduit (23) est ouvert au niveau d'une première bouche (24) qui s'étend de manière sensiblement longitudinale et qui est agencé pour la fixation d'une seringue d'aspiration.

9. Dispositif médical selon la revendication 7 ou 8, dans lequel le conduit (23) est ouvert au niveau d'une seconde bouche (25) qui s'étend, d'une manière incidente par rapport à la direction longitudinale et qui est agencé pour l'entrée d'un fil-guide.

10. Dispositif médical selon l'une quelconque des revendications précédentes, dans lequel des moyens de maintien temporaire sont prévus au niveau de l'extrémité avant (4) du corps tubulaire pour maintenir temporairement la glissière (20) afin de gêner le coulissement de la glissière par rapport au corps tubulaire, lorsque l'aiguille est déplacée dans la position de fonctionnement.

11. Dispositif médical selon la revendication 10, dans lequel les moyens de maintien temporaire comprennent un élément de friction agencé entre le corps tubulaire et la glissière.

12. Dispositif médical selon la revendication 10, dans lequel le corps tubulaire a une fente longitudinale (7) dans laquelle la glissière est mise en prise de manière coulissante, la fente étant fermée au niveau de son extrémité avant (7a) et les moyens de maintien temporaire comprennent un siège (28) formé au niveau de l'extrémité avant de la fente et des moyens (30, 31) pour le couplage par encliquetage entre la glissière et le siège.

13. Dispositif médical selon la revendication 12, dans lequel, au niveau de la fente (7), le corps tubulaire est abaissé par rapport aux zones (2a) du corps tubulaire qui encadrent la fente.

14. Dispositif médical selon l'une quelconque des revendications précédentes, dans lequel la glissière est mobile dans une première position de non fonctionnement dans laquelle la glissière peut être déplacée à nouveau vers la position de fonctionnement, et dans une seconde position de non fonctionnement qui est irréversible et dans laquelle la glissière est ancrée de manière fixe au corps tubulaire afin d'empêcher tout mouvement possible de la glissière vers la position de fonctionnement.

15. Dispositif médical selon la revendication 14, dans lequel :
la glissière comprend une partie interne (21) logée dans le corps tubulaire et une partie externe (22) faisant saillie du corps tubulaire,
on prévoit, sur le corps tubulaire, au niveau de l'extrémité à distance de l'aiguille, un élément de butée (8) capable d'arrêter le déplacement de la glissière lorsque cette dernière est déplacée dans la seconde position de non fonctionnement et l'empêcher de sortir du corps tubulaire,
on étend, à partir de la partie interne de la glissière dans la direction opposée à l'aiguille, un élément d'ancrage (40) capable de se mettre en prise de manière irréversible sur le corps tubulaire (2) ou sur l'élément de butée (8) lorsque la glissière est déplacée dans la seconde position de non fonctionnement contre l'élément de butée.

16. Dispositif médical selon la revendication 14 ou 15, dans lequel on prévoit des moyens de verrouillage amovible pour maintenir la glissière dans la première position de non fonctionnement.

17. Dispositif médical selon la revendication 16, dans lequel, on prévoit, sur le corps tubulaire et sur la glissière, des évidements (35, 36) respectifs qui sont capables de s'aligner lorsque la glissière est déplacée dans la première position de non fonctionnement et les moyens de verrouillage amovible comprennent une broche (38) pouvant se mettre en prise de manière amovible dans les évidements alignés.

18. Dispositif médical selon la revendication 17, dans lequel les évidements comprennent une première paire d'encoches (35) parallèles à l'axe mineur (B) et formées dans le corps tubulaire sur le côté opposé à l'axe majeur (A), et une seconde paire d'encoches (36) parallèles à l'axe mineur et formées dans la glissière du côté opposé à l'axe majeur, et les moyens de verrouillage amovible comprennent une fourche (37), dont les dents (38) forment des broches respectives capables de se mettre en prise dans les encoches lorsque la glissière est dans la première position de non fonctionnement.
